# FASCICULE DE BREVET EUROPEEN

(11) **EP 1 003 537 B1**
(45) Date de publication et mention de la délivrance du brevet: **14.11.2001**
(21) Numéro de dépôt: 98941539.3
(22) Date de dépôt: 04.08.1998
(51) Int. Cl.: A61K 35/78

(54) **COMPOSITION COMPRENANT DE LA PROPOLIS ET AU MOINS UNE HUILE ESSENTIELLE**
PROPOLIS UND MINDESTENS EIN ÄTHERISCHES ÖL ENTHALTENDE ZUSAMMENSETZUNG
COMPOSITION COMPRISING PROPOLIS AND AT LEAST AN ESSENTIAL OIL

(30) Priorité: 11.08.1997 FR 9710414; 19.02.1998 FR 9802027
(43) Date de publication de la demande: 31.05.2000
(73) Titulaire: Morice, André Pierre, 56100 Lorient (FR)
(72) Inventeur: Morice, André Pierre, 56100 Lorient (FR)
(74) Mandataire: Ballot, Paul
(86) Numéro de dépôt international: FR9801739
(87) Numéro de publication internationale: WO9907396

(56) Documents cités:
- EP-A- 0 747 057
- WO-A-97/02040
- DATABASE WPI Section Ch, Week 9325 Derwent Publications Ltd., London, GB; Class B04, AN 93-203503 XP002083501 & RO 103 501 A (INST CONTROLUL STAT MEDICAMENTULUI CERC), 25 mars 1992

## Description

La présente invention se rapporte à une composition comprenant de la propolis et au moins une huile essentielle. Elle concerne également des utilisations de cette composition.

La propolis est une substance chimique synthétisée par les abeilles. Elle est présente sur la surface interne des alvéoles de la ruche. La propolis est connue pour ses propriétés antivirales.

Malheureusement la propolis ne présente pas une activité antivirale suffisante vis-à-vis de certains virus, tels que les virus résistant à la chimiothérapie antivirale.

Aussi il subsiste le besoin d'une composition ayant une activité antivirale suffisante et pouvant être utilisée à de très faibles doses.

L'invention a donc pour objet une composition caractérisée en ce qu'elle comprend de la propolis et au moins une huile essentielle.

La composition présente l'avantage d'utiliser à de très faibles doses, deux constituants d'origine naturelle, facilement disponibles, et dont l'association présente une excellente activité antivirale.

Un autre avantage réside dans le fait que la composition permet, en plus d'un traitement antiviral, le traitement d'affections d'origine bactérienne et/ou mycosique. La composition de l'invention présente encore l'intérêt de pouvoir être facilement appliquée sur la peau et/ou les muqueuses ou être ingérée par un vivant.

Un autre objet de l'invention est une utilisation d'une composition, comme agent antiviral, comprenant de la propolis et au moins une huile essentielle.

Un troisième objet de l'invention est une utilisation d'une composition comprenant de la propolis et au moins une huile essentielle pour la fabrication d'un médicament destiné au traitement des affections bactériennes.

Cette composition permet de traiter notamment des affections liées à la présence de virus à ADN nu ( les adénovirus), de virus enveloppés, tels que ceux de l'herpès ou les virus HSV1, ou encore liées à la présence de papilloma virus ou de virus à ARN nu (poliovirus type 2) ou enveloppés.

Un quatrième objet de l'invention est une utilisation d'une composition comprenant de la propolis et au moins une huile essentielle pour la fabrication d'un médicament destiné au traitement des affections mycosiques présentes sur la peau et/ou les muqueuses.

Cette composition permet de traiter notamment des affections liées à la présence de staphylocoques, de streptocoques B hémolytiques, d'entérobactéries (E. Coli), de pyocyamiques (pseudomonas aéruginosa).

Enfin un dernier objet de l'invention est une utilisation d'une composition comprenant de la propolis et au moins une huile essentielle pour la fabrication d'un médicament destiné à la régénération des cellules et des tissus de la peau et/ou des muqueuses.

Après avoir été correctement nettoyée et, donc débarassée de ses impuretés, la propolis est mélangée, à froid, avec l'huile essentielle.

La composition de l'invention comprend une huile essentielle naturelle choisie parmi l'huile d'Origan, d'Origan Carvacrol, de Girofle, de Mélaleuca alternifolia, de Mélaceuca terpinéol, de Verveine odorante, de Sapin baumier, de sapin Baumé, de Sarriette de Provence, de Verveine, et leurs mélanges.

L'huile essentielle est de préférence comprise en une quantité de 0,1% en poids par rapport au poids total de la composition.

La propolis comprend de préférence de 50 à 55% de résines et de baumes, de 30 à 40% de cires, de 5 à 10% d'huiles essentielles, 5% de pollen, 5% de matières organiques et minérales. La propolis est comprise de préférence en une quantité de 10% en poids par rapport au poids total de la composition.

Par ailleurs, la composition selon l'invention peut comprendre au moins un additif choisi parmi les vitamines, les oligo-éléments, le sucre, les enzymes, les alcools, la vaseline.

La composition de l'invention peut se présenter sous différentes formes galéniques telles que sous forme de suppositoire, de pommade, de teinture, d'inhalation, de gélule, de comprimé, de lotion, d'ovule.

La composition de l'invention peut consister en une composition cosmétique et/ou dermatologique pour la peau et/ou les muqueuses.

L'invention va maintenant être illustrée à l'aide des exemples qui suivent et qui ne sont nullement limitatifs.

Les différentes concentrations sont données en pourcentage pondéral.

### EXEMPLE 1

- Propolis 10
- Malaceuca 0,1
- Excipient qsq 100

Cette composition se présente sous forme d'un ovule destiné au traitement d'infections vaginales.

### EXEMPLE 2

- Propolis 10
- Origan carvacrol 0,1
- Mélaceuca terpinéol 0,1
- Sarriette 0,1
- Girofle 0,1
- Sauge 0,1
- Excipient qsq 100
Cette composition se présente sous forme d'une lotion pour traiter des infections bactériennes et mycosiques.

## Revendications

1. Composition comprenant de la propolis et au moins une huile essentielle choisie parmi l'huile d'origan, de girofle, de verveine, de verveine odorante, de sapin baumier, de sapin baumé, de sarriette de Provence, de Melaleuca alternifolia, de Melaleuca terpinéol, et leurs mélanges.

2. Composition selon la revendication 1, **caractérisée en ce qu'**elle comprend de la propolis et au moins deux huiles choisies parmi l'origan, la sarriette de Provence, le Melaleuca alternifolia, le Melaleuca terpinéol, le girofle et leurs mélanges.

3. Composition selon la revendication 2, **caractérisée en ce qu'**elle comprend en outre au moins une huile choisie parmi la sauge, la lavande et leurs mélanges.

4. Composition selon la revendication 3, **caractérisée en ce qu'**elle comprend de la propolis et les huilles essentielles d'origan, de sarriette de Provence, de sauge, de girofle, de lavande et de Melaleuca terpinéol.

5. Composition selon l'une des revendications 1 à 4, **caractérisée en ce que** l'huile essentielle est présente en une quantité de 0,1 % en poids par rapport au poids total de la composition.

6. Composition selon l'une des revendications 1 à 4, **caractérisée en ce que** la propolis comprend :
- de 50 à 55 % de résines et de baumes,
- de 30 à 40 % de cires,
- de 5 à 10 % d'huiles essentielles,
- 5 % de pollen,
- 5 % de matières organiques et minérales.

7. Composition selon l'une des revendications 1, 2, 3 ou 6, **caractérisée en ce que** la propolis est présente en une quantité de 10 % en poids par rapport au poids total de la composition.

8. Composition selon l'une des revendications précédentes, **caractérisée en ce qu'**elle comprend au moins un additif choisi parmi les vitamines, les oligoéléments, les enzymes, le sucre, les alcools, la vaseline.

9. Composition selon l'une des revendications précédentes, **caractérisée en ce qu'**elle se présente sous forme de suppositoire, de pommade, de teinture, d'inhalation, de gélule, de comprimé, de lotion, d'ovule.

10. Composition selon l'une des revendications précédentes, **caractérisée en ce qu'**elle consiste en une composition cosmétique et/ou dermatologique pour la peau et/ou les muqueuses.

11. Utilisation de la composition, telle que définie selon l'une des revendications précédentes, pour la fabrication d'un médicament destiné au traitement des affections virales.

12. Utilisation de la composition, telle que définie selon l'une des revendications précédentes, pour la fabrication d'un médicament destiné au traitement des affections bactériennes.

13. Utilisation de la composition, telle que définie selon l'une des revendications précédentes, pour la fabrication d'un médicament destiné au traitement des affections mycosiques présentes sur la peau et/ou les muqueuses.

14. Utilisation d'une composition comprenant de la propolis et au moins une huile essentielle pour la fabrication d'un médicament destiné à la régénération des cellules et des tissus de la peau et/ou des muqueuses.

## Patentansprüche

1. Zusammensetzung, die Propolis und mindestens ein etherisches Öl enthält, das unter Origanumöl, Nelkenöl, Verbenaöl, Zitronenverbenenöl, Balsamtannenöl, Öl von balsamhaltigen Tannenarten, Pfefferkrautöl aus der Provence, Teebaumöl (Melaleuca alternifolia), Öl von terpineolhaltigen Melaleuca-Arten und deren Gemischen ausgewählt ist.

2. Zusammensetzung nach Anspruch 1, **dadurch gekennzeichnet, daß** sie Propolis und mindestens zwei Öle enthält, die unter Origanumöl, Pfefferkrautöl aus der Provence, Teebaumöl (Melaleuca alternifolia), Öl von terpineolhaltigen Melaleuca-Arten, Nelkenöl und deren Gemischen ausgewählt ist.

3. Zusammensetzung nach Anspruch 2, **dadurch gekennzeichnet, daß** sie ferner mindestens ein Öl enthält, das unter Salbeiöl, Lavendelöl und deren Gemischen ausgewählt ist.

4. Zusammensetzung nach Anspruch 3, **dadurch gekennzeichnet, daß** sie Propolis und die etherischen Öle Origanumöl, Pfefferkrautöl aus der Provence, Salbeiöl, Nelkenöl, Lavendelöl und Öl von terpineolhaltigen Melaleuca-Arten enthält.

5. Zusammensetzung nach einem der Ansprüche 1 bis 4, **dadurch gekennzeichnet, daß** das etherische Öl in einem Mengenanteil von 0,1 Gew.-%, bezogen auf das Gesamtgewicht der Zusammensetzung, vorliegt.

6. Zusammensetzung nach einem der Ansprüche 1 bis 4, **dadurch gekennzeichnet, daß** das Propolis umfaßt:
- 50 bis 55 % Harze und Balsame,
- 30 bis 40 % Wachse,
- 5 bis 10 % etherische Öle,
- 5 % Pollen, und
- 5 % organische und anorganische Substanzen.

7. Zusammensetzung nach einem der Ansprüche 1, 2, 3 oder 6, **dadurch gekennzeichnet, daß** das Propolis in einem Mengenanteil von 10 Gew.-%, bezogen auf das Gesamtgewicht der Zusammensetzung, vorliegt.

8. Zusammensetzung nach einem der vorhergehenden Ansprüche, **dadurch gekennzeichnet, daß** sie mindestens einen Zusatzstoff enthält, der unter den Vitaminen, Spurenelementen, Enzymen, Zuckern, Alkoholen und Vaseline ausgewählt ist.

9. Zusammensetzung nach einem der vorhergehenden Ansprüche, **dadurch gekennzeichnet, daß** sie als Zäpfchen, Salbe, Tinktur, Inhalationsmittel, Gelatinekapsel, Tablette, Lotion oder Vaginalzäpfchen vorliegt.

10. Zusammensetzung nach einem der vorhergehenden Ansprüche, **dadurch gekennzeichnet, daß** es sich um eine kosmetische und/oder dermatologische Zusammensetzung für die Haut und/oder die Schleimhäute handelt.

11. Verwendung der Zusammensetzung nach einem der vorhergehenden Ansprüche zur Herstellung eines Arzneimittels, das für die Behandlung viraler Erkrankungen vorgesehen ist.

12. Verwendung der Zusammensetzung nach einem der vorhergehenden Ansprüche zur Herstellung eines Arzneimittels, das für die Behandlung bakterieller Erkrankungen vorgesehen ist.

13. Verwendung der Zusammensetzung nach einem der vorhergehenden Ansprüche zur Herstellung eines Arzneimittels, das für die Behandlung mykotischer Erkrankungen der Haut und/oder der Schleimhäute vorgesehen ist.

14. Verwendung einer Zusammensetzung, die Propolis und mindestens ein etherisches Öl enthält, zur Herstellung eines Arzneimittels, das für die Regenerierung von Zellen und Gewebe der Haut und/oder der Schleimhäute vorgesehen ist.

## Claims

1. Composition comprising propolis and at least one essential oil chosen from among oregano, clove, vervain, lemon verbena, balsam fir, silver fir, Provence savory, Melaleuca alternifolia, Melaleuca terpineol oils and their mixtures.

2. Composition according to claim 1, **characterised in that** it comprises propolis and at least two oils chosen from among oregano, Provence savory, Melaleuca alternifolia, Melaleuca terpineol, clove and their mixtures.

3. Composition according to claim 2, **characterised in that** it also comprises at least one oil chosen from among sage, lavender and their mixtures.

4. Composition according to claim 3, **characterised in that** it comprises propolis and essential oils of oregano, Provence savory, sage, clove, lavender and Melaleuca terpineol.

5. Composition according to one of claims 1 to 4, **characterised in that** the essential oil is present in a quantity of 0.1% by weight relative to the total weight of the composition.

6. Composition according to one of claims 1 to 4, **characterised in that** the propolis comprises:
- 50-55% resins and balms,
- 30-40% waxes,
- 5-10% essential oils,
- 5% pollen,
- 5% organic and mineral substances.

7. Composition according to one of claims 1, 2, 3 or 6, **characterised in that** the propolis is present in a quantity of 10% by weight relative to the total weight of the composition,

8. Composition according to one of the preceding claims, **characterised in that** it comprises at least one additive chosen from among vitamins, trace elements, enzymes, sugar, alcohols, Vaseline.

9. Composition according to one of the preceding claims, **characterised in that** it is presented in the form of a suppository, ointment, tincture, inhalation, capsule, tablet, lotion, ovule.

10. Composition according to one of the preceding claims, **characterised in that** it consists of a cosmetic and/or dermatological composition for the skin and/or mucous membranes.

11. Use of the composition as defined according to one of the preceding claims for the manufacture of a medicine intended for the treatment of viral conditions.

12. Use of the composition as defined according to one of the preceding claims for the manufacture of a medicine intended for the treatment of bacterial conditions.

13. Use of the composition as defined according to one of the preceding claims for the manufacture of a medicine intended for the treatment of mycotic conditions present on the skin and/or mucous membranes.

14. Use of a composition comprising propolis and at least one essential oil for the manufacture of a medicine intended for the regeneration of cells and tissues of the skin and/or mucous membranes.
